Europäisches Patentamt

European Patent Office

Office européen des brevets

(11) Veröffentlichungsnummer: **0 111 253**

**A2**

(12) **EUROPÄISCHE PATENTANMELDUNG**

(21) Anmeldenummer: **83112040.7**

(22) Anmeldetag: **01.12.83**

(51) Int. Cl.³: **B 01 J 10/00**
**B 01 J 19/00, C 12 M 1/36**
**C 12 N 1/00**

(30) Priorität: **08.12.82 DE 3245312**

(43) Veröffentlichungstag der Anmeldung:
**20.06.84 Patentblatt 84/25**

(84) Benannte Vertragsstaaten:
**AT BE CH DE FR GB IT LI NL SE**

(71) Anmelder: **HOECHST AKTIENGESELLSCHAFT**
**Postfach 80 03 20**
**D-6230 Frankfurt am Main 80(DE)**

(72) Erfinder: **Hofer, Norbert**
**Waldallee 71**
**D-6239 Eppstein/Taunus(DE)**

(72) Erfinder: **Sittig, Wolfgang**
**Sulzbacher Weg 2**
**D-6238 Hofheim am Taunus(DE)**

(72) Erfinder: **Wöhner, Gerhard, Dr.**
**Flörsheimer Strasse 27**
**D-6093 Flörsheim am Main(DE)**

(54) **Verfahren zum Durchführen (bio-)chemischer Reaktionen.**

(57) Wenn bei (bio-) chemischen Reaktionen mehrere Komponenten reagieren, hemmen oft eine oder mehrere dieser Komponenten die gewünschte Reaktion, sobald eine Maximalkonzentration einer solchen Komponente im Reaktionsgemisch überschritten wird. Es ist also Sorge dafür zu tragen, daß der Anteil einer solchen Komponente unterhalb ihrer schädlichen Maximalkonzentration gehalten wird. Zu diesem Zweck wird die Konzentration einer solchen Komponente im Reaktionsgemisch unmittelbar vor Einleiten neuer Reaktionskomponenten in den Reaktor ermittelt und nach Maßgabe des Meßwertes die Zulaufmenge an neuer (n) Komponente (n) in den Reaktor geregelt. Diese Menge soll höchstens 99 % der Menge entsprechen, die während eines Umlaufs des Reaktionsgemisches im Reaktor umgesetzt wird.

EP 0 111 253 A2

0 1 1 i 253

- 1 -

HOECHST AKTIENGESELLSCHAFT    HOE 82/F 247 D.Ph.HS/mk

Verfahren zum Durchführen (bio-)chemischer Reaktionen

Die Erfindung betrifft ein Verfahren zum Durchführen (bio)-chemischer Reaktionen, bei denen mehrere Komponenten miteinander reagieren und bei denen mindestens eine Komponente die gewünschte Reaktion hemmt, sobald sie eine Maximalkonzentration im Reaktionsgemisch überschreitet.

Unter hemmenden Komponenten sollen solche verstanden werden, die bei Überschreiten einer Maximalkonzentration im Reaktionsgemisch zu einem Verschlechtern der Raumzeitausbeute bzw. zu einer Zunahme von unerwünschten Reaktionsprodukten führen und/oder einen während der Reaktion vorhandenen Katalysator vergiften.

Die Aufgabe zu vorliegender Erfindung besteht demnach darin, ein Verfahren zu schaffen, mit dem (bio-)chemische Reaktionen durchgeführt werden können, bei denen die Zulaufmenge an hemmender Komponente mit wenig Aufwand unterhalb einer kritischen Grenzkonzentration gehalten werden kann, bei unveränderter bzw. gesteigerter Raumzeitausbeute an gewünschten Reaktionsprodukten.

Die Aufgabe wird durch ein Verfahren gelöst, das dadurch gekennzeichnet ist, daß das Reaktionsgemisch in einer Schlaufe geführt wird, die Konzentration der die Reaktion hemmenden Komponente in dem Reaktionsgemisch unmittelbar vor Einleiten neuer Komponente in das Reaktionsgemisch gemessen und mit dem Meßwert die Zulaufmenge an neuer Komponente pro Umlauf des Reaktionsgemisches in der Schlaufe so geregelt wird, daß sie höchstens 99 % der Menge entspricht, die während eines Umlaufs des Reaktionsgemisches umgesetzt werden kann.

Dabei kann die Konzentration der die Reaktion hemmenden Komponente im Reaktionsgemisch direkt oder indirekt gemessen werden. Auch kann die die Reaktion hemmende Komponente an mehreren, in Strömungsrichtung hintereinander angeordneten Stellen in das Reaktionsgemisch eingeleitet werden. Dabei ist jedoch darauf zu achten, daß an jeder Einleitstelle nur soviel Komponente zugeführt wird, daß die über alle Einleitstellen pro Umlauf des Reaktionsgemisches zugeführte Menge an Komponente höchstens 99 % der Menge entspricht, die während eines Umlaufs des Reaktionsgemisches umgesetzt werden kann.

Zur Durchführung des Verfahrens eignen sich gleichermaßen mammutpumpen-, flüssigkeitstreibstrahl- und/oder rührergetriebene Schlaufenreaktoren.

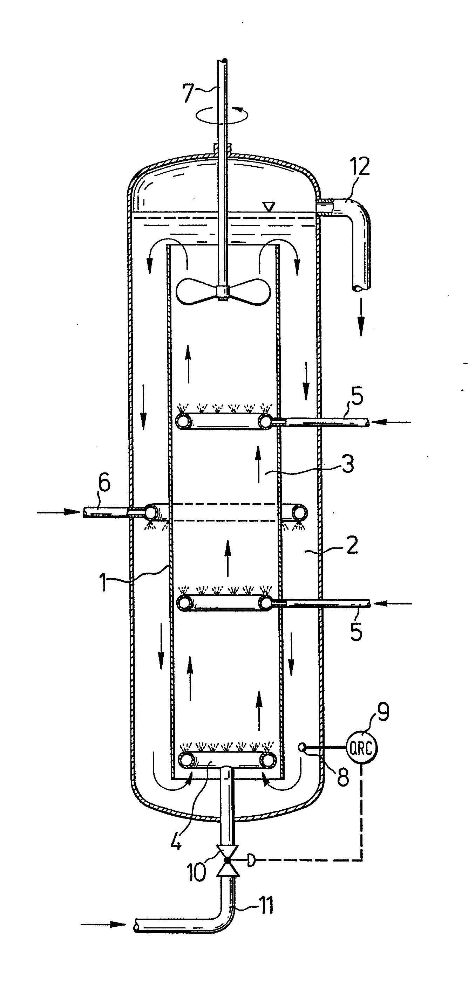

Das Verfahren wird im folgenden anhand der Figur, die lediglich einen Ausführungsweg darstellt, näher erläutert. Der Reaktionsraum wird durch eine Leiteinrichtung 1 in zwei Teilräume 2 und 3 geteilt. Durch den Propellerantrieb 7 wird das Reaktionsgemisch in Bewegung gehalten. Der Propellerantrieb 7 wurde so gewählt, daß Teilraum 2 Abstromraum und Teilraum 3 Aufstromraum für das Reaktionsgemisch ist. Die für die Reaktion erforderlichen Komponenten werden über Einleiteinrichtungen 4, 5 und 6 dem Reaktionsraum zugeführt. Unmittelbar vor der Einleiteinrichtung 4, durch welche die die Reaktion hemmende Komponente in das Reaktionsgemisch eingeleitet wird, ist eine Meßstelle 8 zum Messen der Konzentration der hemmenden Komponente angeordnet. Mit dem Meßwert regelt der Regler 9 das Ventil 10 in der Zuleitung 11 für die hemmende Komponente. Über 12 wird Reaktionsprodukt entnommen.

Beispiel 1

In einem Reaktor nach Figur mit 28 m$^3$ Flüssigkeitsvolumen, einem zylindrischen Teilraum 3 von 1,3 m Durchmesser als Aufstromraum und einem ringförmigen Teilraum 2 mit 1,58 m

0111253

Außen- und 1,32 m Innendurchmesser als Abstromraum wurde Einzellerprotein aus Methanol durch Fermentation hergestellt. Dabei ist das Methanol als eine für die biochemische Reaktion hemmende Komponente zu betrachten, da abhängig von der Konzentration statt Protein unerwünschte Fettsäure produziert wird. Kultursuspension aus Nährsalzen und Methanol, die 10 g/l methylomonas clara enthielt, wurde vorgegeben und durch Einblasen von 1300 m$^3$/h Luft begast. Die Suspension wurde ca. 250 mal/h umgewälzt; und die Methanolzugabe wie üblich entsprechend einem Methanolgehalt von 100 ppm in der Abluft geregelt. Dabei konnte durch Erhöhen oder Erniedrigen der Dosierrate eine Regelgenauigkeit von ± 15 ppm erreicht werden. Nährsalzlösung mit Mikroorganismus wurde kontinuierlich mit 5 m$^3$/h zudosiert und 5,8 m$^3$/h Überlauf kontinuierlich abgenommen. Die abgeernteten getrockneten Zellen des Mikroorganismus enthielten einen Rohfettanteil von 8 bis 10.% und einen Rohproteinanteil von 78 %.

Unter Anwendung der erfindungsgemäßen Verfahrensweise wurde die Regelung der Methanolzugabe durch Messen des Sauerstoffgehaltes im Abstromraum 2 des Reaktors und zwar unmittelbar vor der Einleitstelle 4 vorgenommen. Der $O_2$-Gehalt betrug 0,7 ± 0,2 mg/l. Der in der Abluft meßbare Methanolgehalt betrug 7 ± 2 ppm. Die gewonnene Zellmasse wies einen Proteingehalt von 81 ± 1 % und einen Fettgehalt von weniger als 4 % auf.

Beispiel 2

In einem Reaktor nach der Figur mit 4 m$^3$ Flüssigkeitsvolumen und einem zylinderförmigen Teilraum 3 mit 502 mm Durchmesser als Aufstromraum und einem ringförmigen, konzentrisch angeordneten Teilraum 2 mit 590 mm Außen- und 508 mm Innendurchmesser als Abstromraum wurde ein glykol- und cellulosehaltiges Abwasser mit 4 bis 7 % Natriumchlorid und einem CSB-Wert von 20 g $O_2$/l mit Bakterien

angereichert und mit 130 m$^3$/h Luft begast. Nach Erreichen einer Bakteriendichte von ca. 15 g/l wurde Abwasser nachdosiert. Es ergab sich durch CSB-Schwankungen im Abwasserzulauf sehr schnell eine Behinderung der Abbauleistung der eingesetzten Bakterienkultur, die durch die Messung des $CO_2$-Gehaltes der Abluft nachgewiesen werden konnte. Demenstprechend mußte die Zudosierung von Abwasser immer wieder abgestellt werden, bis der $CO_2$-Gehalt sich erholt hatte. Schließlich stellte sich nach einer Verweilzeit von mehr als 40 Stunden ein Rest CSB-Gehalt von 8 g $O_2$/l ein.

Gemäß der Erfindung wurde eine Messung des Sauerstoffgehaltes der Kulturlösung im Abstromraum 2 des Reaktors kurz oberhalb des Bodens vorgenommen. Die Abwasserdosierung wurde bei Überschreiten eines $O_2$-Gehaltes von 1,5 mg/l bei gleichzeitig niedrigem $CO_2$-Gehalt zurückgenommen bzw. erhöht bei gleichzeitig hohem $CO_2$-Gehalt. Es konnte ein Rest CSB-Gehalt von 6 bis 7 g $O_2$/l bei einer Verweilzeit von 12 Stunden erreicht werden.

Beispiel 3

In einem Schlaufenreaktor gemäß Figur wurde eine Essigsäurewasserlösung mit suspendierten Katalysatorsalzen vorgelegt. In die Lösung wird Ethylen eingeblasen. Die Bewegung der Lösung wird durch Mammutpumpenantrieb erzeugt. Die Reaktionskomponente Sauerstoff wird über drei Gasverteiler 4,5 zugeführt, die im Aufstromraum des Reaktors in unterschiedlicher Höhe angeordnet sind. Über die beiden unteren Sauerstoffzuführungen werden konstante Mengen von ca. 2/3 der Gesamtsauerstoffmenge eingetragen. Über die oberste, in ca. 2/3 der Gesamthöhe angeordnete Zuführung wird soviel Sauerstoff zugeführt, daß der $O_2$-Partialdruck im Abstromraum des Reaktors unmittelbar vor der Umlenkung ins Innenrohr gemessen und die Konzentration bei etwa 0,5 mg Sauerstoff/l gehalten wird. Neben einer besseren Regelbarkeit der Sauerstoffkonzentration im Reaktor konnte auch die Grenzsauerstoffkonzentration von ca. 4 % im Reaktorabgas deutlich unterschritten werden.

Patentansprüche:

1. Verfahren zum Durchführen (bio-)chemischer Reaktionen, bei denen mehrere Komponenten miteinander reagieren und bei denen mindestens eine Komponente die gewünschte Reaktion hemmt, sobald sie eine Maximalkonzentration im Reaktionsgemisch überschreitet, dadurch gekennzeichnet, daß das Reaktionsgemisch in einer Schlaufe geführt wird, die Konzentration der die Reaktion hemmenden Komponente im Reaktionsgemisch unmittelbar vor Einleiten neuer Komponente in das Reaktionsgemisch ermittelt und mit dem Meßwert die Zulaufmenge an neuer Komponente pro Umlauf des Reaktionsgemisches in der Schlaufe so geregelt wird, daß sie höchstens 99 % der Menge entspricht, die während eines Umaufs des Reaktionsgemisches umgesetzt werden kann.

2. Verfahren nach Anspruch 1, dadurch gekennzeichnet, daß die Konzentration der die Reaktion hemmenden Komponente unmittelbar von Einleiten neuer Komponente in das Reaktionsgemisch indirekt gemessen, und der Meßwert zum Regeln der Zulaufmenge an neuer Komponente benutzt wird.

3. Verfahren nach Anspruch 1, dadurch gekennzeichnet, daß die die Reaktion hemmende Komponente an mehreren in Strömungsrichtung hintereinander angeordneten Stellen in das Reaktionsgemisch eingeleitet wird, wobei an jeder Einleitstelle nur soviel Komponente zugeführt wird, daß die über alle Einleitstellen pro Umlauf des Reaktionsgemisches zugeführte Menge an Komponente höchstens 99 % der Menge entspricht, die während eines Umlaufs des Reaktionsgemisches umgesetzt werden kann.